# EUROPÄISCHE PATENTANMELDUNG

(11) **EP 1 035 105 A1**
(43) Veröffentlichungstag der Anmeldung: **13.09.2000**
(21) Anmeldenummer: 00104024.5
(22) Anmeldetag: 26.02.2000
(51) Int. Cl.: C07C 69/62, C08F 2/50

(54) **Estergruppen enthaltende Iodoniumsalze und ihre Verwendung zur Strahlenhärtung kationisch härtender Massen**

(30) Priorität: 11.03.1999 DE 19910780
(71) Anmelder: Goldschmidt AG, 45127 Essen (DE)
(72) Erfinder: Oestreich, Sascha, Dr., 45279 Essen (DE)

(57) **Zusammenfassung**

Die Erfindung betrifft Estergruppen enthaltende Iodoniumsalze mit verringerter Kristallisationsneigung sowie deren Verwendung zur Strahlenhärtung kationisch härtender Massen.

## Beschreibung

Die vorliegende Erfindung betrifft Estergruppen enthaltende Iodoniumsalze mit verringerter Kristallisationsneigung sowie deren Verwendung zur Strahlenhärtung kationisch härtender Massen.

Die kationische Photopolymerisation ist ein schneller, effizienter und umweitschonender Weg, um kationisch polymerisierbare Monomere zu härten. Besonders effiziente Photoinitiatoren stellen Diaryliodonium- (I) und Triarylsulfoniumsalze (II) dar.

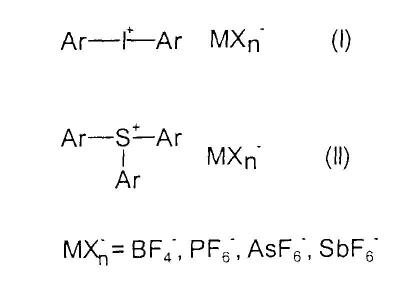

Insbesondere Diaryliodoniumsalze (I) sind aus der Patentliteratur bekannt (DE-A-25 18 639, US-A-4 279 717, EP-A-0 334 056, EP-B-0 618 919) und werden als Photoinitiatoren zur Polymerisation von kationisch polymerisierbaren Substanzen verwendet. Die kationisch polymerisierbaren Substanzen sind aber unpolar bis wenig polar, besonders wenn die polymerisierbaren Gruppen in Organopolysiloxanen enthalten sind. Es wird daher beim Zusatz dieser Photoinitiatoren sehr häufig beobachtet, daß, abhängig von der Struktur der Zubereitung, die Mischbarkeit und Löslichkeit der Photoinitiatoren begrenzt ist. Daher werden die Arylreste solcher Oniumsalze oft mit Alkylketten substituiert, um die Löslichkeit in Organopolysiloxanen zu erhöhen (US-A-4 310 469 und US-A-4 374 066).

Diaryliodoniumsalze, wie sie in US-A-5 073 643 beschrieben werden, sind ebenfalls durch Alkylketten hydrophob modifiziert. Zusätzlich tragen sie jedoch eine Hydroxylgruppe. Diese führt zu einer mangelhaften Mischbarkeit mit Organopolysiloxanen. Schon nach kurzer Zeit werden eine Trübung und ein Niederschlag beobachtet.

Aufgrund ihrer Inhomogenität härten solche Beschichtungen bei UV-Bestrahlung nur schlecht aus. Es ist aber auch möglich, daß durch die Inhomogenität schon während der Applikation einer dünnen Schicht auf einem Substrat massive Oberflächenstörungen (Krater, Runzeln, Stippen etc.) auftreten.

Bei Hydroxylgruppen tragenden Iodoniumsalzen, wie sie in US-A-5 073 643 beschrieben werden, wird die schlechte Löslichkeit in unpolaren Medien auf die hohe Kristallisationsneigung zurückgeführt. Das besondere Komplexbildungsverhalten der Hydroxylgruppen tragenden Iodoniumsalze der allgemeinen Formel (III) führt zu einer starken Kristallisationsneigung der Verbindungen (A. Kunze, U. Müller, K. Tittes, J.P. Fouassier, F. Morlet-Savary, J. Photochemistry and Photobiology A: Chemistry, 110, 115-122 (1997):

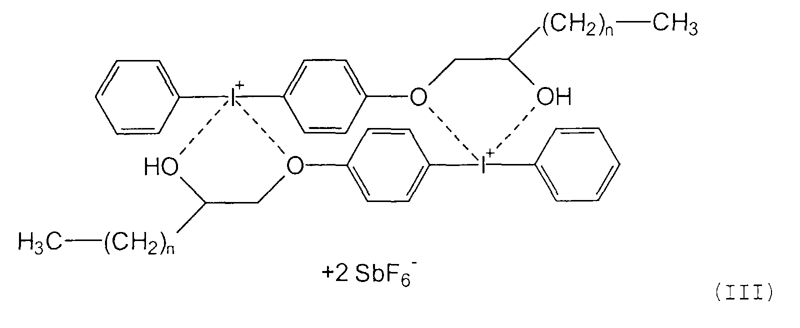

Die beiden Sauerstoffatome des Moleküls wirken als Liganden für ein zweites Iodoniumion. Durch dieses Aggregationsverhalten wird die Ausbildung von Kristallen gefördert.

Bei der Herstellung dieser Salze ist die starke Kristallisationsneigung durchaus erwünscht, da dadurch die Verbindungen durch einfache Umkristallisation in hoher Reinheit als Pulver gewonnen werden können. Auf diese Weise sind sie einfach und kostengünstig herstellbar. Solche Iodoniumsalze sind unter dem Namen CD-1012 von Sartomer kommerziell erhältlich.

Die hohe Kristallisationsneigung wirkt sich allerdings negativ aus, wenn die lodoniumsalze in unpolaren Medien, wie Organopolysiloxanen, gelöst werden sollen. Hier sind sie entweder unlöslich oder es bildet sich schon nach kurzer Zeit ein fester Niederschlag.

Der vorliegenden Erfindung liegt die Aufgabe zugrunde, Hydroxylgruppen tragende Iodoniumsalze auf eine besonders kostengünstige, einfache Art so zu modifizieren, daß die Kristallisationsneigung stark herabgesetzt wird, eine gute Verträglichkeit mit Epoxygruppen aufweisenden Organopolysiloxanen hergestellt wird und die entstehenden Verbindungen hydrolysestabil sind.

Die vorgenannte Aufgabe wird gelöst durch Iodoniumsalze der allgemeinen Formel (IV) 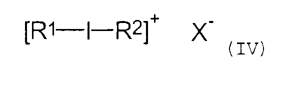 wobei
- I: Iod ist,
- X⁻: ein Anion eines komplexen Metallsalzes oder einer starken Säure ist,
- R¹: der Rest 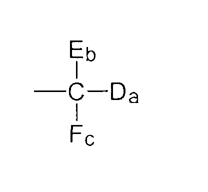 ist, wobei
- C: ein einwertiger aromatischer Kohlenwasserstoffrest mit 6 bis 14 Kohlenstoffatomen je Rest oder ein einwertiger, mindestens ein Sauerstoff- und/oder Schwefelatom enthaltender aromatischer Kohlenwasserstoffrest mit 5 bis 15 Ringatomen je Rest ist,
- a: 1, 2 oder 3,
- b: 0, 1 oder 2,
- c: 0, 1 oder 2 sind,
- D, E und F: jeweils Substituenten von C sind, wobei
- D: ein Rest der Formel 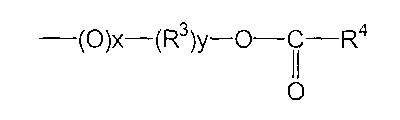 ist, wobei
- x: 0 oder 1,
- y: 0 oder 1 sind,
- R³: ein linearer oder verzweigter, zweiwertiger Kohlenwasserstoffrest mit 1 bis 40 Kohlenstoffatomen je Rest, der gegebenenfalls durch mindestens ein Sauerstoffatom und/oder ein Schwefelatom und/oder eine Carboxylgruppe unterbrochen sein kann, ist,
- R⁴: ein einwertiger linearer, verzweigter und/oder cyclischer Alkyl-, Aryl-, Haloalkyl-, Haloaryl- und/oder Alkoxyradikalrest mit 1 bis 40 Kohlenstoffatomen ist,
- E: ein Rest der Formel ―O―R⁵
- F: ein Rest der Formel ―R⁶
- R²: ein Rest der Formel 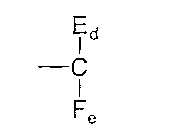 ist, wobei
- R⁵: ein einwertiger Kohlenwasserstoffrest mit 1 bis 18 Kohlenstoffatomen je Rest ist, der gegebenenfalls durch mindestens ein Sauerstoffatom unterbrochen sein kann,
- R⁶: ein einwertiger Kohlenwasserstoffrest mit 1 bis 18 Kohlenstoffatomen je Rest ist, der gegebenenfalls durch mindestens ein Sauerstoffatom unterbrochen sein kann,
- d: 0, 1 oder 2 und
- e: 0, 1 oder 2 sind.

Überraschenderweise wurde gefunden, daß durch die Veresterung von Hydroxylgruppen enthaltenden Iodoniumsalzen die Kristallisationsneigung erheblich herabgesetzt, die Verträglichkeit mit Epoxygruppen aufweisenden Organopolysiloxanen wesentlich verbessert und die Hydrolysestabilität gegenüber Si-O-C-Bindungen enthaltenden Iodoniumsalzen erheblich erhöht werden kann.

Bevorzugte Beispiele für aromatische Kohlenwasserstoffreste C sind der Phenyl-, Naphthyl- und Anthrylrest.

Bevorzugte Beispiele für mindestens ein Sauerstoff- und/oder Schwefelatom enthaltende aromatische Kohlenwasserstoffreste C sind der 2-Furyl-, 3-Furyl-, 2-Thienyl- und 3-Thienylrest.

Bevorzugte Beispiele für den zweiwertigen Kohlenwasserstoffrest R³, der durch mindestens ein Sauerstoffatom und/oder Schwefelatom und/oder eine Carboxylgruppe unterbrochen sein kann, sind -CH₂-, -CH₂-CH₂-, -CH₂-CH₂-O-CH₂-CH₂-, -CH₂-CH(CH₃)-, -CH₂-CH(CH₂CH₃)-, -CH₂-CH((CH₂)₃CH₃)-, -CH₂-CH((CH₂)₁₁CH₃)-, -CH₂-CH((CH₂)₁₃CH₃)-.

Bevorzugte Beispiele für den einwertigen linearen, verzweigten und/oder cyclischen Alkyl-, Aryl-, Haloalkyl-, Haloaryl-und/oder Alkoxyradikalrest R⁴ sind Alkylreste, wie der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentylrest; Hexylreste, wie der n-Hexylrest, wie der Cyclohexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest; Dodecylreste, wie der n-Dodecylrest und Octyldecylreste, wie der n-Octadecylrest; Arylreste, wie der Phenyl-, Naphthyl- und Anthrylrest; Haloalkylreste, wie der Chlormethyl-, Trichlormethyl-, Trifluormethyl-, Pentafluorethyl- und Heptafluorpropylrest; Haloarylreste, wie der Bromphenyl-, Chlorphenyl-, Fluorphenyl-, Pentafluorphenyl- und Trifluormethylphenylrest; Alkoxyreste, wie der Methoxy-, Ethoxy-, Propoxy- und Butoxyrest.

Die vorgenannten Beispiele für den Rest R⁴ gelten in vollem Umfang auch für die Reste R⁵ und R⁶.

Beispiele für durch mindestens ein Sauerstoffatom und/oder Schwefelatom unterbrochene Kohlenwasserstoffe R⁴, R⁵ und R⁶ sind -CH₂-CH₂-O-CH₃, -CH₂-CH₂O-CH₂CH₃ und -CH₂-CH₂-S-CH₂CH₃.

Bevorzugte Beispiele für Reste D sind

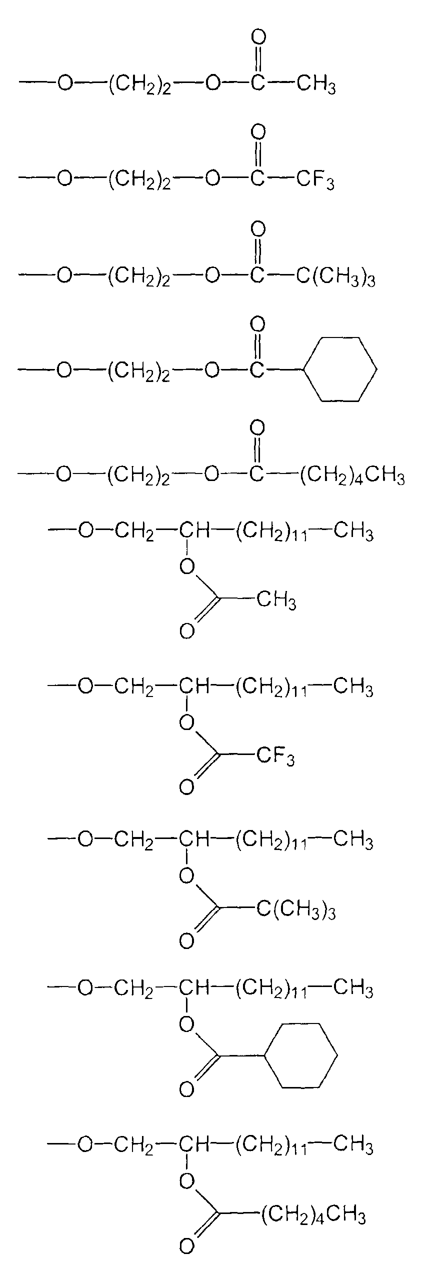

Bevorzugte Beispiele für Reste E sind der Methoxy-, Ethoxy- und der n-Butoxyrest.

Bevorzugte Beispiele für Reste F sind der Methyl-, Ethyl-, Propyl-, 2-Methylpropyl- und der n-Butylrest.

Bevorzugte Beispiele für Reste R¹ sind

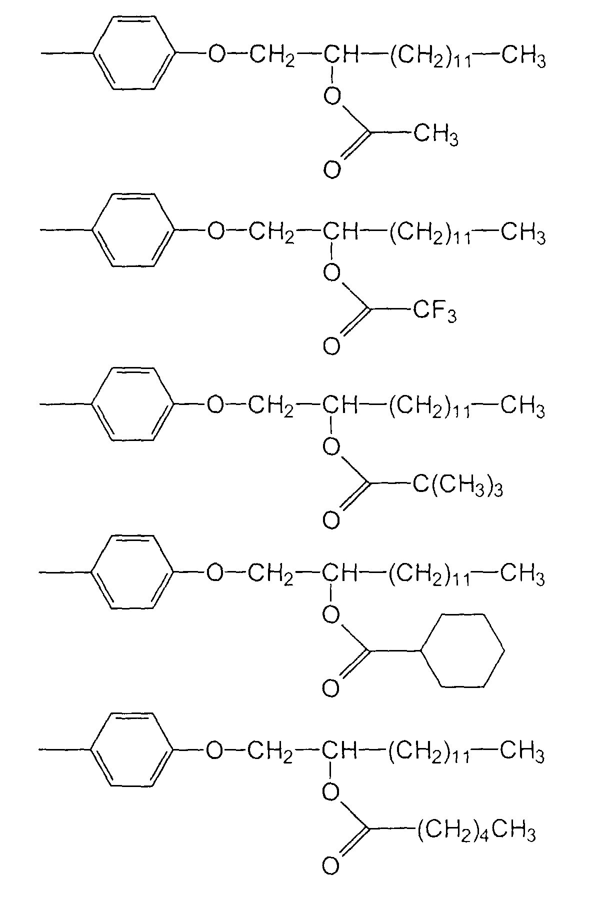

Bevorzugte Beispiele für Reste R² sind der Phenyl-, 4-Methylphenyl-, 3-Methoxyphenyl- und 4-Methoxyphenylrest.

Bevorzugte Beispiele für Anionen X⁻ eines komplexen Metallsalzes oder einer starken Säure sind Tosylat, SbF₆⁻, PF₆⁻, BF₄⁻, F₃CSO₃⁻, F₃CCO₂⁻, AsF₆⁻, ClO₄⁻, HSO₄⁻. Starke Säuren im Sinne der vorliegenden Erfindung umfassen insbesondere starke Brönstedt-Säuren.

Bevorzugt sind als Iodoniumsalze mit verminderter Kristallisationsneigung solche der allgemeinen Formel (V) 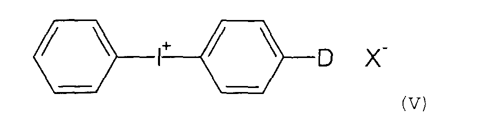 wobei D und X⁻ die bereits angegebene Bedeutung haben.

Besonders bevorzugt sind Iodoniumsalze mit verminderter Kristallisationsneigung der Formel (VI) 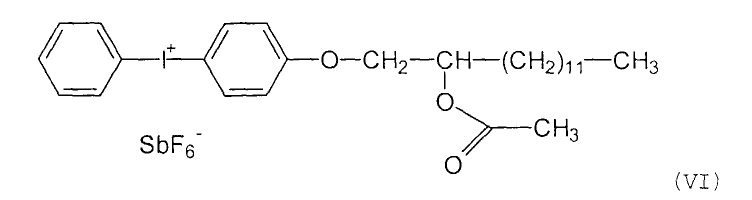 und der Formel (VII)

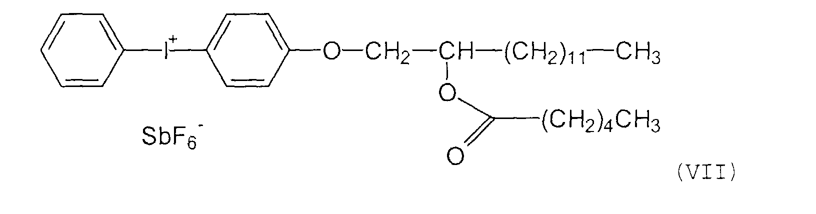

Die Kristallisationsneigung der so modifizierten Iodoniumsalze ist gegenüber dem eingangs erwähnten Stand der Technik wesentlich herabgesetzt. Beispielsweise stellt das Iodoniumsalz (VII) bei Raumtemperatur eine viskose Flüssigkeit dar, während das vergleichbare Hydroxylgruppen enthaltende Iodoniumsalz mit der nachfolgenden Formel (VIII) 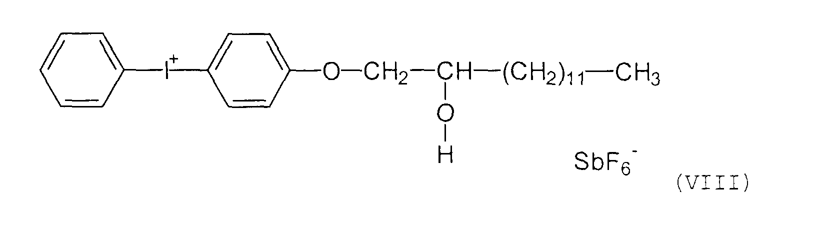 ein Pulver mit einem Schmelzpunkt von 91 °C darstellt, welches unter dem Handelsnamen CD-1012 von Sartomer erhältlich ist.

Ebenso ist die Löslichkeit der erfindungsgemäßen Iodoniumsalze in unpolaren Medien, wie n-Alkanen oder Siloxanen, wesentlich großer als die der vergleichbaren Hydroxylgruppen enthaltenden Iodoniumsalze.

Beispielsweise ist das Iodoniumsalz der allgemeinen Formel (VII) unbegrenzt in Toluol löslich. Dagegen ist das vergleichbare, Hydroxylgruppen enthaltende Iodoniumsalz der allgemeinen Formel (VIII) unlöslich in Toluol und zeigt keine Mischbarkeit mit Epoxygruppen enthaltenden Organopolysiloxanen.

Gegenstand der Erfindung ist weiterhin ein Verfahren zur Herstellung der Iodoniumsalze mit verminderter Kristallisationsneigung. Erfindungsgemäße Iodoniumsalze werden beguemerweise durch eine Vielfalt von Routen hergestellt wie unten angegeben. Hydroxylgruppen enthaltende Iodoniumsalze können mit einer Säure, einem Säurechlorid, einem Säureanhydrid oder einem Ester reagieren. 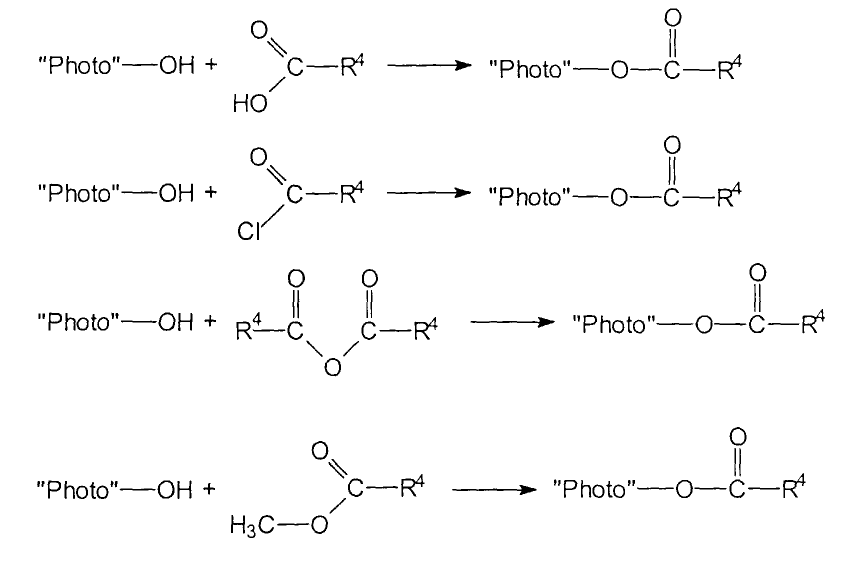 wobei "Photo" einen zu modifizierenden Photoinitiatorrest bezeichnet.

Das erfindungsgemäße Verfahren ist dadurch gekennzeichnet, daß ein Hydroxylgruppen enthaltendes Iodoniumsalz mit der allgemeinen Formel (IX) 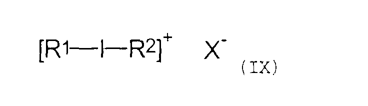 wobei
- I: Iod ist,
- X⁻: ein Anion eines komplexen Metallsalzes oder einer starken Säure ist,
- R¹: ein Rest 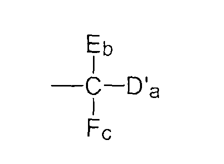 ist, worin
- C: ein einwertiger aromatischer Kohlenwasserstoffrest mit 6 bis 14 Kohlenstoffatomen je Rest oder ein einwertiger, mindestens ein Sauerstoff- und/oder Schwefelatom enthaltender aromatischer Kohlenwasserstoffrest mit 5 bis 15 Ringatomen je Rest ist,
- a: 1, 2 oder 3,
- b: 0, 1 oder 2,
- c: 0, 1 oder 2 sind,
- D', E und F: jeweils Substituenten von C sind, wobei
- D': ein Rest der Formel ―(O)x―(R³)y―OH ist, wobei
- x: 0 oder 1,
- y: 0 oder 1 sind,
- R³: ein linearer oder verzweigter, zweiwertiger Kohlenwasserstoffrest mit 1 bis 40 Kohlenstoffatomen je Rest, der gegebenenfalls durch mindestens ein Sauerstoffatom und/oder ein Schwefelatom und/oder eine Carboxylgruppe unterbrochen sein kann, ist,
- E: ein Rest der Formel ―O―R⁵
- F: ein Rest der Formel ―R⁶
- R²: ein Rest der Formel 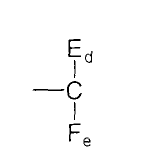 sind, wobei
- R⁵: ein einwertiger Kohlenwasserstoffrest mit 1 bis 18 Kohlenstoffatomen je Rest ist, der gegebenenfalls durch mindestens ein Sauerstoffatom unterbrochen sein kann,
- R⁶: ein einwertiger Kohlenwasserstoffrest mit 1 bis 18 Kohlenstoffatomen je Rest ist, der gegebenenfalls durch mindestens ein Sauerstoffatom unterbrochen sein kann,
- d: 0, 1 oder 2 und
- e: 0, 1 oder 2 sind,
umgesetzt wird mit einer Carbonsäure, einem Carbonsäurehalogenid, einem Carbonsäureanhydrid und/oder einem Carbonsäureester.

Bevorzugt werden als Hydroxylgruppen Iodoniumsalze der allgemeinen Formel (X) 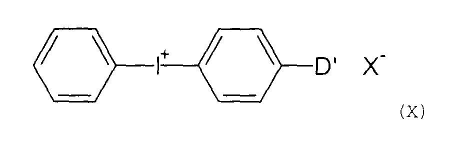 wobei D' und X⁻ die bereits angegebene Bedeutung haben.

Besonders bevorzugte Ausgangsmaterialien sind Iodoniumsalze der nachfolgenden Formel (XI):

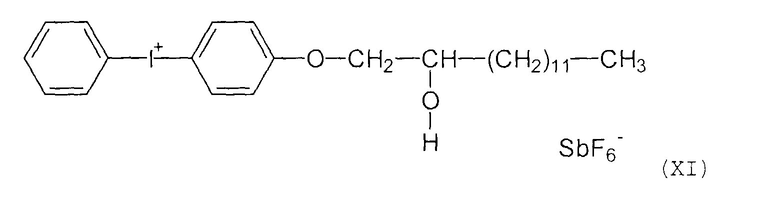

Beispiele für Carbonsäuren, die mit Hydroxylgruppen tragenden Iodoniumsalzen umgesetzt werden können, sind solche mit der allgemeinen Formel (XII) 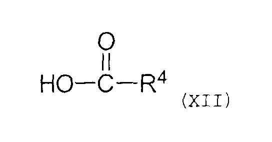 wobei
- R⁴: ein einwertiger linearer, verzweigter und/oder cyclischer Alkyl-, Aryl-, Haloalkyl-, Haloaryl- und/oder Alkoxyradikalrest mit 1 bis 40 Kohlenstoffatomen ist.

Besonders bevorzugte Carbonsäuren, die mit Hydroxylgruppen tragenden Iodoniumsalzen umgesetzt werden können, sind Acrylsäure, Arachinsäure, Benzoesäure, 4-Biphenylessigsäure, 4-Brombenzoesäure, 2-Brombuttersäure, Bromessigsäure, 2-Brompropionsäure, Buttersäure, Butylformiat, Chloressigsäure, 4-Chlorbenzoesäure, Chlormethylbuttersäure, 4-Chlorphenylessigsäure, 3-Chlorpropionsäure, 5-Chlorvaleriansäure, Crotonsäure, Cyclohexancarbonsäure, 3-Cyclohexylpropionsäure, Decansäure, 2,6-Dichlorbenzoesäure, Dichloressigsäure, 3,4-Difluorbenzoesäure, 2,6-Dimethoxybenzoesäure, 3,3-Dimethylbuttersäure, Diphenylessigsäure, Essigsäure, Ethoxyessigsäure, 2-Ethylbuttersäure, 2-Ethylhexansäure, 4-Fluorbenzoesäure, 4-Fluorphenylessigsäure, Heptafluorbuttersäure, Hexansäure, Isobuttersäure, Isovaleriansäure, Laurinsäure, Methacrylsäure, 4-Methoxybenzoesäure, Methoxyessigsäure, 4-Methoxyphenylessigsäure, 4-Methoxyzimtsäure, 4-Methylbenzoesäure, 2-Methylvaleriansäure, Myristinsäure, Naphthalin-2-carbonsäure, 2-Naphthylessigsäure, Octansäure, Ölsäure, Önanthsäure, Palmitinsäure, Pelargonsäure, Pentadecafluoroctansäure, Pentadecansäure, Pentafluorbenzoesäure, 2,2,3,3,3-Pentafluoropropionsäure, 4-Pentinsäure, 4-Phenylbuttersäure, Phenylessigsäure, 3-Phenylpropionsäure, Pivalinsäure, Propionsäure, 2-Propylvaleriansäure, Stearinsaure, Trichloressigsäure, Tridecansäure, Trifluoressigsäure, Trifluormethylbenzoesäure, 2,4,6-Trimethylbenzoesäure, Undecansäure, Valeriansäure und/oder Zimtsäure.

Beispiele für Carbonsäurehalogenide, die mit Hydroxylgruppen tragenden Iodoniumsalzen umgesetzt werden können, sind solche mit der allgemeinen Formel (XIII) 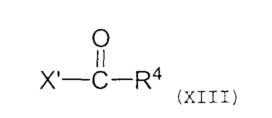 wobei
- R⁴: ein einwertiger linearer, verzweigter und/oder cyclischer Alkyl-, Aryl-, Haloalkyl-, Haloaryl- und/oder Alkoxyradikalrest mit 1 bis 40 Kohlenstoffatomen ist und
- X': ein Element aus der Reihe Fluor, Chlor, Brom, Iod ist.

Besonders bevorzugte Carbonsäurehalogenide, die mit Hydroxylgruppen tragenden Iodoniumsalzen umgesetzt werden können, sind Acetylbromid, Acetylchlorid, Acrylsäurechlorid, Benzoylchlorid, Benzoylbromid, 4-Brombenzoylchlorid, Buttersäurechlorid, Butylchlorformiat, Chloracetylchlorid, 4-Chlorbenzoylchlorid, 4-Chlorbuttersäurechlorid, 4-Chlorphenylessigsäurechlorid, 3-Chlorpropionsäurechlorid, Crotonsäurechlorid, Cyclohexancarbonsäurechlorid, Decansäurechlorid, Dichloracetylchlorid, 3,3-Dimethylbuttersäurechlorid, 4-Fluorbenzoylchlorid, Heptafluorbuttersäurechlorid, Hexansäurechlorid, Isobuttersäurechlorid, Isobutylchlorformiat, Isovaleriansäurechlorid, Laurinsäurechlorid, 4-Methoxybenzoylchlorid, 4-Mechylbenzoylchlorid, Methylchlorformiat, Myristinsäurechlorid, Naphthalin-2-carbonsäurechlorid, Octansäurechlorid, Ölsäurechlorid, Palmitinsäurechlorid, Pelargonsäurechlorid, Pentafluorobenzoesäurechlorid, Phenylacetylchlorid, Phenylchlorformiat, 3-Phenylpropionsäurechlorid, Pivalinsäurechlorid, Propionsäurechlorid, Stearinsäurechlorid, Trichloressigsäurechlorid, Trichlormethylchlorformiat, Trifluoressigsäurechlorid, Valeriansäurechlorid und/oder Zimtsäurechlorid.

Beispiele für Carbonsäureanhydride, die mit Hydroxylgruppen tragenden Iodoniumsalzen umgesetzt werden können, sind solche mit der allgemeinen Formel (XIV) 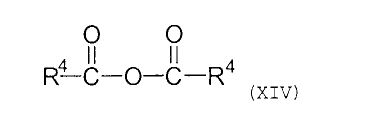 wobei
- R⁴: ein einwertiger linearer, verzweigter und/oder cyclischer Alkyl-, Aryl-, Haloalkyl-, Haloaryl- und/oder Alkoxyradikalrest mit 1 bis 40 Kohlenstoffatomen ist.

Besonders bevorzugte Carbonsäureanhydride, die mit Hydroxylgruppen tragenden Iodoniumsalzen umgesetzt werden können, sind Acetanhydrid, Buttersäureanhydrid, Chloressigsäureanhydrid, Crotonsäureanhydrid, Dichloressigsäureanhydrid, Essigsäureanhydrid, Heptafluorbuttersäureanhydrid, Hexansäureanhydrid, Isobuttersäureanhydrid, Isovaleriansäureanhydrid, Pivalinsäureanhydrid, Propionsäureanhydrid, Trufluoressigsäureanhydrid und/oder Valeriansäureanhydrid.

Beispiele für Carbonsäureester, die mit Hydroxylgruppen tragenden Iodoniumsalzen umgesetzt werden können, sind solche mit der allgemeinen Formel (XV) 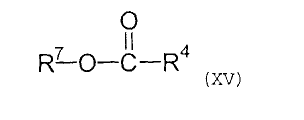 wobei
- R⁴ und R⁷: jeweils unabhängig ausgewählt sind und einen einwertigen, linearen, verzweigten und/oder cyclischen Alkyl-, Aryl-, Haloalkyl-, Haloaryl- und/oder Alkoxyradikalrest mit 1 bis 40 Kohlenstoffatomen bedeuten.

Besonders bevorzugte Carbonsäureester, die mit Hydroxylgruppen tragenden Iodoniumsalzen umgesetzt werden können, sind Ethylacetat, Ethylacrylat, Ethylbenzoat, Ethylbutyrat, Ethylchloratetat, Ethylchlorformiat, Ethylcrotonat, Ethyldecanoat, Ethyldichloracetat, Ethyllaurat, Ethylmethacrylat, Ethyl-2-methylbutyrat, Ethylmyristat, Ethyloctanoat, Ethylpalmitat, Ethylphenylacetat, Ethylpivalat, Ethylpropionat, Ethylstearat, Ethyltrifluoracetat, Ethylvalerat, Isopropylbutyrat, Methylacetat, Methylacrylat, Methylarachidat, Methylbenzoat, Methylbutyrat, Methylchloracetat, Methylcrotonat, Methyldichloracetat, Methylisobutyrat, Methyllinoleat, Methylmethacrylat, Methyloctanoat, Methyloleat, Methylpalmitat, Methylpropionat, Methyltrichloracetat, Methyltrifluoracetat, Methylvalerat und/oder Propylpropionat.

Die Iodoniumsalze sind lichtempfindlich und zersetzen sich beispielsweise bei Bestrahlung mit Ultraviolettlicht nach einem mehrstufigen Mechanismus, der im Buch "UV-Curing: Science & Technology" von P. Pappas auf S. 34 beschrieben ist. Als aktives Endprodukt dieser Photolyse wird die entstandene Brönstedt-Säure, beispielsweise HPF₆, HAsF₆, angesehen, die ihrerseits die Polymerisation von kationisch polymerisierbaren Substanzen, wie Epoxiden oder Vinylethern, startet.

Die erfindungsgemäßen Iodoniumsalze eignen sich als Photoinitiatoren für die Polymerisation von kationisch polymerisierbaren organischen Substanzen, wie Epoxiden, Vinylethern, Epoxygruppen enthaltenden Organopolysiloxanen, Alkenyloxygruppen, wie Vinyloxygruppen oder Propenyloxygruppen, enthaltenden Organopolysiloxanen und Olefinen. Solche Substanzen sind beispielsweise in US-A-5 057 549, DE-A-40 02 922 sowie in den eingangs genannten Patentschriften enthalten.

### Ausführungsbeispiele

### Beispiel 1

10 g eines handelsüblichen Photoinitiators gemäß der Formel (XI) werden in 30 g Acetylchlorid gelöst und bei 40 °C im Wasserbad erwärmt. Das Gemisch wird 1 h gerührt. Nach der Reaktion wird das überschüssige Acetylchlorid destillativ entfernt. Zurück bleibt ein braunes, viskoses Produkt.

### Beispiel 2

10 g eines handelsüblichen Photoinitiators gemäß der Formel (XI) werden in 30 g Essigsäureanhydrid gelöst und bei 40 °C im Wasserbad erwärmt. Das Gemisch wird 1 h gerührt. Nach der Reaktion wird das überschüssige Essigsäureanhydrid destillativ entfernt. Zurück bleibt ein gelbes, viskoses Produkt.

### Beispiel 3

10 g eines handelsüblichen Photoinitiators gemäß der Formel (XI) werden in 30 g Propionsäureanhydrid gelöst und bei 40 °C im Wasserbad erwärmt. Das Gemisch wird 1 h gerührt. Nach der Reaktion wird das überschüssige Propionsäureanhydrid destillativ entfernt. Zurück bleibt ein gelbes, viskoses Produkt.

### Beispiel 4

10 g eines handelsüblichen Photoinitiators gemäß der Formel (XI) werden in 30 g Pivalinsäureanhydrid gelöst und bei 40 °C im Wasserbad erwärmt. Das Gemisch wird 1 h gerührt. Nach der Reaktion wird das überschüssige Pivalinsäureanhydrid destillativ entfernt. Zurück bleibt ein farbloses, viskoses Produkt.

### Vergleich 1

Die Löslichkeit der erfindungsgemäßen Iodoniumsalze wird mit der Löslichkeit der hydroxylgruppentragenden Iodoniumsalze verglichen. Die Ergebnisse sind in Tabelle 1 zusammengefaßt:

**Tabelle 1**

| Photoinitiator | Löslichkeit in Toluol |
|---|---|
| Formel (XI) | unlöslich |
| Bsp. 1 | klar löslich |
| Bsp. 2 | klar löslich |
| Bsp. 3 | klar löslich |
| Bsp. 4 | klar löslich |

Der Löslichkeitsunterschied zwischen dem Hydroxylgruppen enthaltenden Photoinitiator mit der Formel (XI) und den erfindungsgemäßen, Estergruppen enthaltenden Photoinitiatoren zur kationischen Härtung ist beträchtlich.

### Vergleich 2

Die Verminderung der Kristallisationsneigung läßt sich besonders deutlich anhand des Schmelzpunktes der Verbindungen aufzeigen.

**Tabelle 2**

| Photoinitiator | Schmelzpunkt |
|---|---|
| Formel (XI) | 91 °C |
| Bsp. 1 | viskoses Öl |
| Bsp. 2 | viskoses Öl |
| Bsp. 3 | viskoses Öl |
| Bsp. 4 | viskoses Öl |

Es zeigt sich, daß durch die Modifikation der Schmelzpunkt und damit die Kristallisationsneigung erheblich herabgesetzt ist.

### Vergleich 3

Die verbesserte hydrolytische Stabilität der erfindungsgemäßen, Estergruppen enthaltenden Iodoniumsalze läßt sich am besten im Vergleich mit dem Si-O-C-Bindungen enthaltenden Iodoniumsalz mit der Formel (XVI) zeigen.

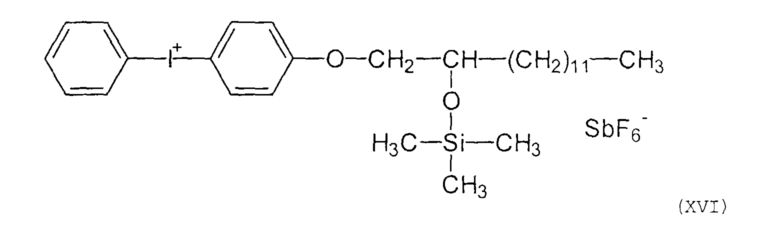

Die Ergebnisse sind in Tabelle 3 zusammengefaßt. Angegeben ist die Zeit bis zur Bildung sichtbarer Niederschläge bzw. Kristalle des hydrolytischen Spaltprodukts mit der Formel (XI):

**Tabelle 3**

| Photoinitiator | Stabilität in Toluol (50 % w/w) | Stabilität in 1-Butanol (50 % w/w) |
|---|---|---|
| Formel (XVI) | 21 d | 2 d |
| Bsp. 1 | > 90 d | 11 d |
| Bsp. 2 | > 90 d | 14 d |
| Bsp. 3 | > 90 d | > 30 d |
| Bsp. 4 | > 90 d | > 90 d |

### Versuche zur kationischen Photopolymerisation

Um die Aktivität, der in den Beispielen 1 - 4 dargestellten Verbindungen, in der kationischen Photopolymerisation zu überprüfen, wurden 98 Teile eines cycloaliphatischen Epoxysiloxans mit einem Epoxywert von 3,5 % und einer Viskosität von 125 mPas mit jeweils 2 Teilen Photoinitiator gemischt.

Die Mischungen wurden dann mit einer Technikumsbeschichtungsmaschine, ausgerüstet mit einem Fünf-Walzen-Antragswerk, auf eine Standard-OPP Folie (30µm) aufgetragen. Das Auftragsgewicht betrug 0,5 - 1 g/m². Die Beschichtungen wurden anschließend mit einer mikrowellenangeregten UV-Lampe (Fusion, 120 W/cm) bei einer Bahngeschwindigkeit von 20 m/min ausgehärtet.

Direkt nach dem Passieren der UV-Lampe wurde bestimmt, ob die Beschichtungen zu einem nicht mehr klebrigen Film ausgehärtet sind. Die Ergebnisse sind in der folgenden Tabelle zusammengefaßt:

| Photo-initiator | Aussehen der Mischung | Aushärtung nach der UV-Lampe |
|---|---|---|
| Formel (XI) | trübe, kristalliner Niederschlag | ungenügend; trüber, klebriger Film; Kristalle sichtbar |
| Bsp. 1 | klar | gute Aushärtung; klarer, nicht mehr klebriger Film |
| Bsp. 2 | klar | gute Aushärtung; klarer, nicht mehr klebriger Film |
| Bsp. 3 | opak | gute Aushärtung; klarer, nicht mehr klebriger Film |
| Bsp. 4 | klar | gute Aushärtung; klarer, nicht mehr klebriger Film |

Es zeigt sich, daß die Unterschiede im Härtungsverhalten zwischen dem Hydroxylgruppen enthaltenden Photoinitiator mit der Formel (XI) und den enfindungsgemäßen, Estergruppen enthaltenden Photoinitiatoren beträchtlich sind.

## Patentansprüche

1. Iodoniumsalze der allgemeinen Formel (IV) 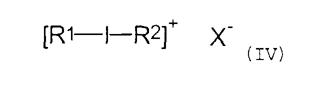 wobei
I Iod ist,
X⁻ ein Anion eines komplexen Metallsalzes oder einer starken Säure ist,
R¹ der Rest 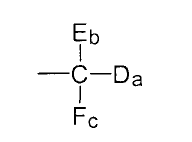 ist, wobei
C ein einwertiger aromatischer Kohlenwasserstoffrest mit 6 bis 14 Kohlenstoffatomen je Rest oder ein einwertiger, mindestens ein Sauerstoff- und/oder Schwefelatom enthaltender aromatischer Kohlenwasserstoffrest mit 5 bis 15 Ringatomen je Rest ist,
a 1, 2 oder 3,
b 0, 1 oder 2,
c 0, 1 oder 2 sind,
D, E und F jeweils Substituenten von C sind, wobei
D ein Rest der Formel 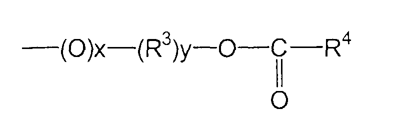 ist, wobei
x 0 oder 1,
y 0 oder 1 sind,
R³ ein linearer oder verzweigter, zweiwertiger Kohlenwasserstoffrest mit 1 bis 40 Kohlenstoffatomen je Rest, der gegebenenfalls durch mindestens ein Sauerstoffatom und/oder ein Schwefelatom und/oder eine Carboxylgruppe unterbrochen sein kann, ist,
R⁴ ein einwertiger linearer, verzweigter und/oder cyclischer Alkyl-, Aryl-, Halonlkyl-, Haloaryl- und/oder Alkoxyradikalrest mit 1 bis 40 Kohlenstoffatomen ist,
E ein Rest der Formel 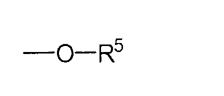
F ein Rest der Formel 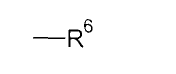
R² ein Rest der Formel 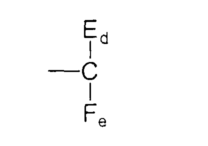 ist, wobei
R⁵ ein einwertiger Kohlenwasserstoffrest mit 1 bis 18 Kohlenstoffatomen je Rest ist, der gegebenenfalls durch mindestens ein Sauerstoffatom unterbrochen sein kann,
R⁶ ein einwertiger Kohlenwasserstoffrest mit 1 bis 18 Kohlenstoffatomen je Rest ist der gegebenenfalls durch mindestens ein Sauerstoffatom unterbrochen sein kann,
d 0, 1 oder 2 und
e 0, 1 oder 2 sind.

2. Iodoniumsalze gemäß Anspruch 1 der allgemeinen Formel V 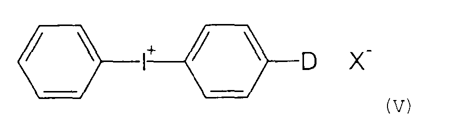 wobei D und X⁻ die bereits angegebene Bedeutung haben.

3. Iodoniumsalze gemäß den Ansprüchen 1 und 2 der allgemeinen Formel (XVII) 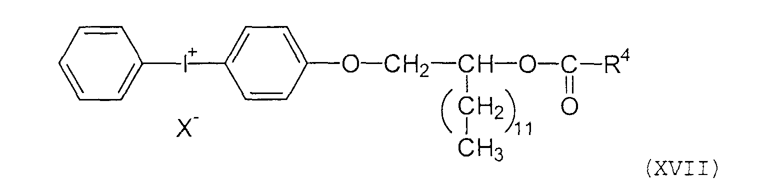 wobei X⁻ und R⁴ die bereits angegebene Bedeutung haben.

4. Iodoniumsalze gemäß den Ansprüchen 1, 2 und 3, dadurch gekennzeichnet, daß X⁻ ausgewählt ist aus der Gruppe von Tosylat, SbF₆⁻, PF₆⁻, BF₄⁻, F₃CSO₃⁻, F₃CCO₂⁻, AsF₆⁻, ClO₄⁻, HSO₄⁻.

5. Iodoniumsalze der Formel

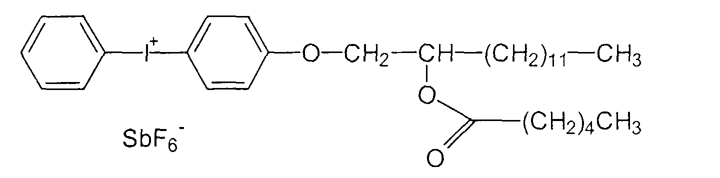

6. Iodoniumsalze der Formel

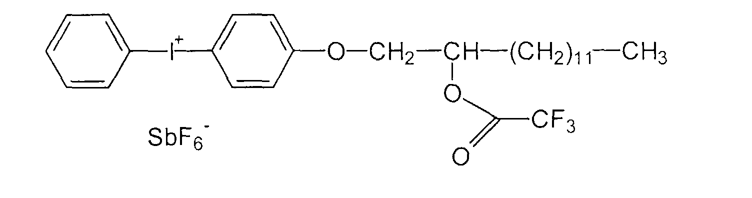

7. Iodoniumsalze der Formel

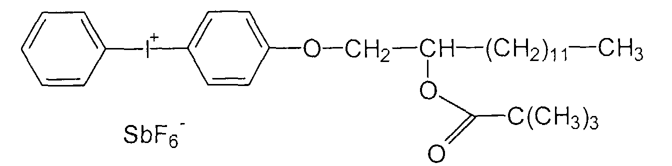

8. Iodoniumsalze der Formel

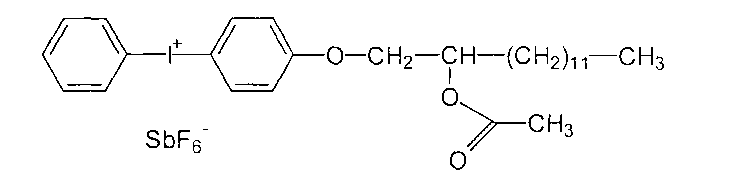

9. Verwendung von Iodoniumsalzen gemäß den Ansprüchen 1 bis 8 als Photoinitiator zur Polymerisation von kationisch polymerisierbaren Substanzen, insbesondere Epoxiden, Vinylethern, Epoxygruppen enthaltenden Organopolysiloxanen, Alkenyloxygruppen enthaltenden Polysiloxanen und Olefinen.
